# EUROPEAN PATENT SPECIFICATION

(11) **EP 1 929 936 B1**
(45) Date of publication and mention of the grant of the patent: **19.11.2014**
(21) Application number: 06024998.4
(22) Date of filing: 04.12.2006
(51) Int. Cl.: A61B 5/00

(54) **Protecting means in particular for non-invasive procedures**
Schutzvorrichtung insbesondere für nicht-invasive Verfahren
Moyen de protection surtout pour des procédures non-invasives

(43) Date of publication of application: 11.06.2008
(73) Proprietor: ETHICON ENDO-SURGERY, INC., Cincinnati, OH 45242-2839 (US)
(72) Inventor: D'Arcangelo, Michele, 00142 Roma (IT); Pastorelli, Alessandro, 00136 Roma (IT); Bilotti, Federico, 04011 Aprilia, Latina (IT); Popovic, Drago, 4567 Queensland (AU)
(74) Representative: Leihkauf, Steffen Falk

(56) References cited:
- WO-A-2006/094243
- WO-A1-2005/079683
- GB-A- 2 071 502
- JP-A- 7 079 909
- US-A- 5 716 321
- US-A1- 2002 013 601

## Description

FIELD OF THE INVENTION

The subject of the present invention is a protecting means suitable for being used during non-invasive procedures, for example in endoluminal or laparoscopic procedures. Moreover the present invention relates to a method for protecting tissues during non-invasive procedures, for example endoluminal or laparoscopic procedures. According to one specific example the present method may be applied for protecting a hollow body organ during endoluminal procedures.

BACKGROUND OF THE INVENTION

The advantages of endoluminal procedures in respect of traditional surgery are well known; they allow a less invasive approach with therefore a lower level of risk and a quicker and safer convalescence.

However always new needs arise while improving these advantageous procedures. In particular the development of always new therapeutic devices that allow treatment of different pathologies stresses new drawbacks.

Indeed whenever an endoluminal procedure is performed, the risk of perforation of the hollow organ is always a possibility. Perforation may occur during device introduction through the natural orifice, device navigation and during the therapeutic procedure itself. When a perforation occurs, the patient is immediately sent to surgery to obviate. GB2071502 discloses a surgical retractor, JP07079909 discloses a guide for endoscopy, WO2005/079683 discloses an endoscopic tissue stabilization device and US2002/013601 discloses a cavity enlarger apparatus which protect hollow organs while an endoscopic therapeutic instrument is introduced.

US 6, 974, 441 shows an inflatable intraluminal molding device for positioning, isolating and/or identifying relevant segments of a hollow viscous in which a sleeve comprises a plurality of independent balloon sections. Each balloon section is inflated by an individual air line. This structure has its better performance for isolating segments of a hollow viscous inflating selectively one or more balloon sections. However it has not been explicitly designed for protecting the tissues of the viscous and for forming a flexible structure suitable for absorbing the forces exerted by instruments introduced in the sheath.

The problem upon which the present invention is based is that of proposing a protecting means suitable for being used during non-invasive procedures, for example in endoluminal or laparoscopic procedures, which has structural and functional characteristics such as to satisfy the above-mentioned requirements and, at the same time, to overcome the disadvantages mentioned with reference to the prior art.

### SUMMARY OF THE INVENTION

This problem is solved by a protecting means according to Claim 1. In particular this problem is solved by a protecting means comprising a sheath extending from a distal end to a proximal end, in which the sheath comprises at least one inflatable channel having an opening and being suitable for receiving a fluid (liquid or gas) injected through said opening whereby said fluid injected into the inflatable channel through said opening inflates at least one portion of the sheath. The inflatable channel comprises at least two annular inflatable segments along the length of the sheath and at least one longitudinal inflatable segment connecting said two annular inflatable segments. The protecting means further comprises an outer sleeve and an inner sleeve forming a gap that comprises said plurality of longitudinal inflatable segments and said plurality of annular inflatable segments in form of a frame of the sheath within the gap between the outer and inner sleeve. These advantageous features allow to introduce the protecting means in a deflated state in order to minimize transversal dimension and then to inflate it injecting a fluid in the at least one inflatable channel in order to protect the body tissues.

### BRIEF DESCRIPTION OF THE DRAWINGS

Further characteristics and the advantages of the protecting means and the method according to the invention will become clear from the following description of a preferred embodiment thereof, provided by way of nonlimiting example with reference to the appended drawings, in which:

FIG. 1 shows a side view of the protecting means according to the present invention, in a inflated state;

FIG. 2 shows a perspective view of an enlarged detail of fig. 1;

FIG. 3 shows a perspective view of a step of the method according to the present invention in which the protecting means is shown in a deflated state and over an endoscope within a hollow body organ;

FIG. 4 shows a perspective view of an enlarged detail of fig. 3 in which the protecting means has been inflated;

FIG: 5 shows a perspective view of an enlarged detail of the protecting means.

DETAILED DESCRIPTION OF THE INVENTION

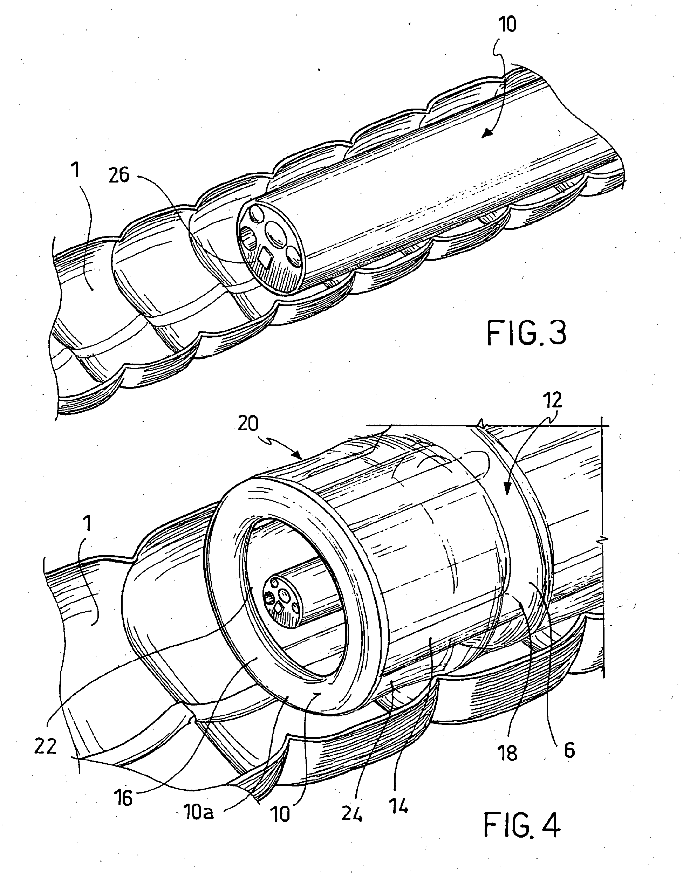

With reference to the drawings, a protecting means suitable for being used during non-invasive procedures, for example in endoluminal or laparoscopic procedures, has been shown. With reference to figures 3 and 4, an endoluminal procedure within a hollow body organ 1 has been shown.

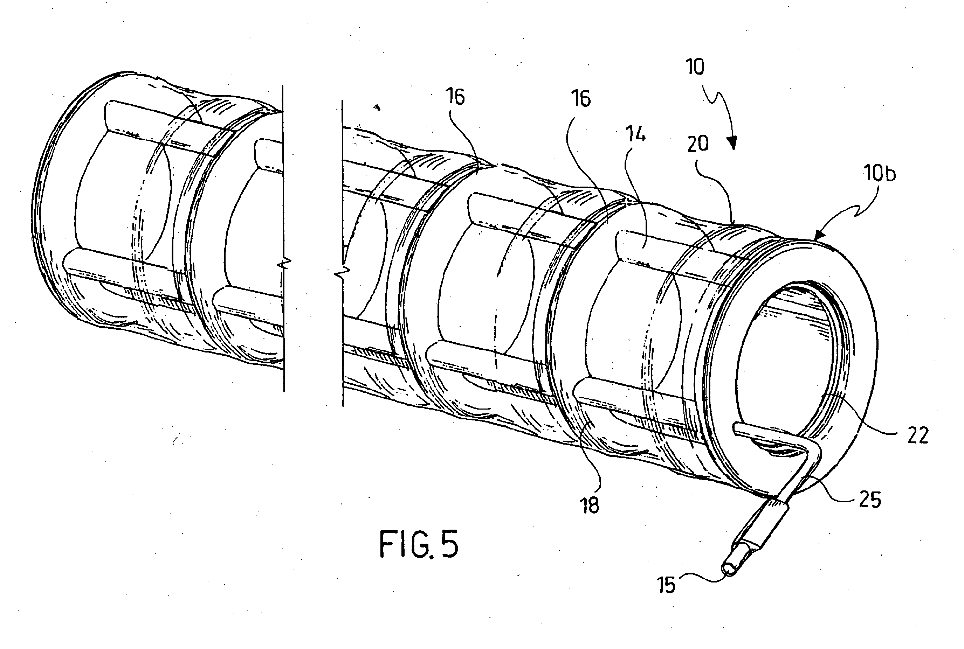

The protecting means comprises a sheath 10 extending from a distal end 10a to a proximal end 10b (FIG. 5). The sheath comprises at least one inflatable channel 12 having an opening 15 and being suitable for receiving a fluid (liquid or gas) injected through said opening whereby said fluid injected into the inflatable channel through said opening inflates at least one portion of the sheath.

As shown in figures, said inflatable channel 12 comprises a plurality of longitudinal inflatable segments 14 and a plurality of annular inflatable segments 16 connected to the longitudinal inflatable segments. The plurality of longitudinal inflatable segments 14 and the plurality of annular inflatable segments 16 are disposed in shape of a frame, each annular inflatable segment 16 crossing one or more longitudinal inflatable segments 14.

In other words the plurality of longitudinal inflatable segments 14 and the plurality of annular inflatable segments 16 are a frame or net of the sheath.

In particular one annular inflatable segment of said plurality of annular inflatable segments is located at the distal end 10a of the sheath.

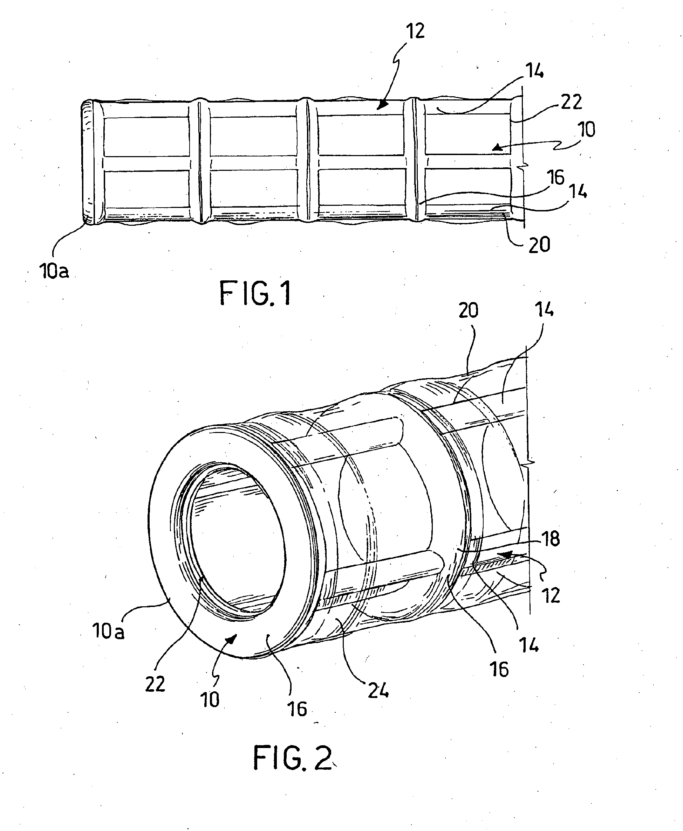

Figures 1 and 2 show the detailed structure of this latter embodiment of the sheath 10 and in particular of the longitudinal and annular inflatable segments 14, 16 that, in case of endoluminal procedure in an hollow organ 1, keep the hollow organ expanded and prevent kinking of the sheath itself.

Advantageously the inflatable segments 14, 16 of the sheath form a flexible structure suitable for absorbing the forces exerted by instruments introduced in the sheath.

According to the invention, shown in the annexed drawings, the protecting means further comprises an outer sleeve 20 and an inner sleeve 22 forming a gap 24 that comprise the plurality of longitudinal inflatable segments 14 and the plurality of annular inflatable segments 16 connected to the longitudinal inflatable segments.

According to a possible embodiment of the invention, the protecting means further comprises injecting means suitable for being connected to the inflatable channel through said opening. In particular said injecting means comprises means for injecting a saline solution or other bio-compatible fluid (gas or liquid). Fig. 5 shows an enlarged detail of the proximal end 10b in which a tube 25 is connected to the at least one inflatable channel. The tube 25 ends with the opening 15. An injecting means, for example a syringe, is connected to the tube 25 through said opening 15 in order to inject the fluid in the inflatable channel.

According to a possible embodiment of the invention, the protecting means further comprises a deploying device 26 suitable for retaining and inserting said sheath in a deflated state up the desired location of the body. In particular the deploying device comprises an elongate body and the sheath is suitable for being placed and fit over said elongate body in the deflated state (fig. 3). In this case the elongate body is transversally (radially) smaller than the sheath in an inflated state, whereby the elongate body runs within the sheath in the inflated state.

More particularly, as shown in the figures 3 and 4, the deploying device comprises an endoscope and the sheath is suitable for being placed and fit over said endoscope in the deflated state. In this case the endoscope is transversally (radially) smaller than the sheath in an inflated state, whereby the endoscope runs within the sheath in the inflated state.

Therefore, once at the desired location the inflated sheath separates from the elongate body (scope) and deploys in site, particularly in the hollow organ 1 in case of endoluminal procedures.

The present disclosure relates also to a method for protecting tissues during non-invasive procedures, for example endoluminal or laparoscopic procedures comprising the steps of:

providing a sheath 10 extending from a distal end 10a to a proximal end 10b, the sheath comprising at least one inflatable channel 12 having an opening 15 and being suitable for receiving a fluid (liquid or gas) injected through said opening,

introducing the sheath 10 in a deflated state up to the desired location of the body,

injecting the fluid in the inflatable channel 12 in order to inflate the sheath,

introducing at least an instrument through the inflated sheath.

More particularly said sheath is placed over an elongate body 24 before introducing it up to the desired location of the body. In this case the elongate body 24 runs within the sheath 10 in the inflated state as it is transversally smaller than an inner transversal dimension of the sheath in an inflated state.

According to an example, during endoluminal procedures in a hollow body organ 1, said sheath 10 is placed over an endoscope before introducing it up to the desired location of the hollow body organ. In this case the endoscope runs within the sheath in the inflated state it is transversally smaller than an inner transversal dimension of the sheath in an inflated state.

According to a possible example of the method, said sheath is inflated via saline solution or other biocompatible fluid.

In case of an endoluminal procedure within a hollow body organ, the sheath 10 is introduced transanally or transorally up to desired location of the hollow body organ.

Finally said sheath 10 is deflated before removing from the body.

Summarising, the preferred way to deploy the sheath 10 in an endoluminal procedure would be through an endoscope and the step could be the followings:

placing the sheath over the endoscope in the deflated state,

introducing the endoscope and the sheath transanally or transorally up to desired site,

inflating the sheath, preferably via saline solution,

introducing endoscopic instruments to allow therapeutic procedure,

deflating and removing the sheath.

The present method is particularly suitable for being performed in the colon pathway. As a preferred solution, figures 3 and 4 show the sheath carried by a colonscope through the colon pathway.

The present invention allows to protect tissues during endoluminal procedures. In particular the present invention allows to protect the inner walls of a hollow body organ in case of endoluminal procedures, such as introduction of an endoluminal stapler to treat suspicious lesion of the colon pathway. The protection is assured prior to introduction of therapeutic endoscopic instrument (i.e. staplers, anastomotic rings, suturing devices).

More particularly the flexible embodiment of the sheath allows to absorb the forces induced by instruments (in particular endoscopic instruments) which would otherwise be exerted on the hollow organ.

Moreover the protection is obtained by means of a simple structure such as an inflatable protective sheath to be deployed inside the body (in particular inside the hollow body organ).

The advantageous choice of an inflatable sheath allows to insert the sheath in a deflated state up the desired location of the body and then to inflate the sheath to the desired shape in a simple and quick way, for example by means of saline solution. In this way it is possible to minimize the increase of diameter during insertion of endoscope and sheath.

Moreover in particular in case of endoluminal procedure, besides providing protection of the hollow body organ, the membrane expands the lumen diameter in order to create more space for passage of endoscopic instruments. This is particularly important in the colon; where the sigmoid bend, the splenic and hepatic flexures can have tight bending radiuses and can compromise passage of endoluminal devices. In this particular case, it is preferable that the sheath expands to an inner diameter of approx 5 cm adhering to the colonic wall. In this way the sheath can act as a protective cushion for endoscopic instruments that need to reach the desired site.

The structure is completed by the inner and outer sleeves which are preferably made by a transparent material in order to allow to visualize the site, in particular the wall of the hollow organ with the endoscope if required.

Naturally variants and/or additions may be provided for the embodiment described and illustrated above.

In order to satisfy contingent and specific requirements, an expert in the art may apply to the above-described preferred embodiment of the protecting means many modifications, adaptations without, however, departing from the scope of the following claims.

## Claims

1. Protecting means in particular for non-invasive procedures, comprising a sheath (10) extending from a distal end (10a) to a proximal end (10b), in which the sheath comprises at least one inflatable channel (12) having an opening (15) and being suitable for receiving a fluid injected through said opening whereby said fluid injected into the inflatable channel through said opening inflates at least one portion of the sheath,
in which said inflatable channel comprises a plurality of annular inflatable segments (16) along the length of the sheath (10) and a plurality of longitudinal inflatable segments (14) connected to said plurality of annular inflatable segments (16) by means of cross-connections (18),
**characterized in that** the protecting means comprises an outer sleeve (20) and an inner sleeve (22) forming a gap (24) that comprises said plurality of longitudinal inflatable segments (14) and said plurality of annular inflatable segments (16) in form of a frame of the sheath (10) within the gap (24) between the outer and inner sleeve (20, 22).

2. Protecting means according to claim 1, in which one annular inflatable segment of said plurality of annular inflatable segments (16) is located at the distal end (10a) of the sheath (10).

3. Protecting means according to claim 1, in which the outer sleeve (20) is made by a transparent material.

4. Protecting means according to claim 1, in which the inner sleeve (22) is made by a transparent material.

5. Protecting means according to one of preceding claims, further comprising injecting means suitable for being connected to the inflatable channel through said opening (15).

6. Protecting means according to claim 5, in which said injecting means comprising means for injecting a saline solution or other bio-compatible fluid.

7. Protecting means according to one of preceding claims, further comprising a deploying device (26) suitable for retraining and inserting said sheath (10) in a deflated state up the desired locution of the body.

8. Protecting means according to claim 7, in which the deploying device (26) comprises an elongate body and the sheath (10) is suitable for being placed and fit over said elongate body in the deflated state.

9. Protecting means according to claim 8, in which the elongate body is transversally smaller than the sheath (10) in an inflated state, whereby the elongate body runs within the sheath in the inflated state.

10. Protecting means according to claim 8 or 9, in which the elongate body is an endoscope.

## Patentansprüche

1. Schutzvorrichtung insbesondere für nicht-invasive Verfahren, umfassend eine Hülle (10), die sich von einem distalen Ende (10a) zu einem proximalen Ende (10b) erstreckt, wobei die Hülle mindestens einen aufblasbaren Kanal (12) umfasst, der eine Öffnung (15) aufweist und zur Aufnahme eines durch die Öffnung injizierten Fluids geeignet ist, wodurch das in den aufblasbaren Kanal durch die Öffnung injizierte Fluid zumindest einen Teil der Hülle aufbläst, wobei der aufblasbare Kanal eine Vielzahl von ringförmigen aufblasbaren Segmenten (16) entlang der Länge der Hülle (10) und eine Vielzahl von longitudinalen aufblasbaren Segmenten (14) umfasst, die mittels Kreuzverbindungen mit der Vielzahl von ringförmigen aufblasbaren Segmenten (16) verbunden sind,
**dadurch gekennzeichnet, dass** die Schutzvorrichtung eine äußere Hülse (20) und eine innere Hülse (22) umfasst, die einen Spalt (24) bilden, der die Vielzahl von longitudinalen aufblasbaren Segmenten (14) und die Vielzahl von ringförmigen aufblasbaren Segmenten (16) in Form eines Gerüsts der Hülle (10) in dem Spalt (24) zwischen der äußeren und inneren Hülse (20, 22) umfasst.

2. Schutzvorrichtung nach Anspruch 1, wobei ein ringförmiges aufblasbares Segment der Vielzahl von ringförmigen aufblasbaren Segmenten (16) an dem distalen Ende (10a) der Hülle (10) angeordnet ist.

3. Schutzvorrichtung nach Anspruch 1, wobei die äußere Hülse (20) aus einem transparenten Material hergestellt ist.

4. Schutzvorrichtung nach Anspruch 1, wobei die innere Hülse (22) aus einem transparenten Material hergestellt ist.

5. Schutzvorrichtung nach einem der vorangehenden Ansprüche, ferner umfassend ein Injiziermittel, das zur Verbindung mit dem aufblasbaren Kanal durch die Öffnung (15) geeignet ist.

6. Schutzvorrichtung nach Anspruch 5, wobei das Injiziermittel ein Mittel zum Injizieren einer Salzlösung oder eines anderen biokompatiblen Fluids umfasst.

7. Schutzvorrichtung nach einem der vorangehenden Ansprüche, ferner umfassend eine Ausbringeinrichtung (26), die zum Halten und Einführen der Hülle (10) in einem entleerten Zustand bis zum gewünschten Ort des Körpers geeignet ist.

8. Schutzvorrichtung nach Anspruch 7, wobei die Ausbringeinrichtung (26) einen länglichen Körper umfasst und die Hülle (10) zum Platzieren und Sitzen über dem länglichen Körper in dem entleerten Zustand geeignet ist.

9. Schutzvorrichtung nach Anspruch 8, wobei der längliche Körper transversal kleiner als die Hülle (10) in einem aufgeblasenen Zustand ist, wodurch der längliche Körper in der Hülle in dem aufgeblasenen Zustand läuft.

10. Schutzvorrichtung nach Anspruch 8 oder 9, wobei der längliche Körper ein Endoskop ist.

## Revendications

1. Moyen de protection, en particulier pour des procédures non invasives, comprenant une gaine (10) qui s'étend à partir d'une extrémité distale (10a) jusqu'à une extrémité proximale (10b), dans lequel la gaine comprend au moins un canal gonflable (12) comportant une ouverture (15) et qui est approprié pour recevoir un fluide qui est injecté à travers ladite ouverture, moyennant quoi ledit fluide injecté dans le canal gonflable à travers ladite ouverture gonfle au moine une partie de la gaine,
dans lequel ledit canal gonflable comprend une pluralité de segments gonflables annulaires (16) le long de la longueur de la gaine (10), et une pluralité de segments gonflables longitudinaux (14) qui sont connectés à ladite pluralité de segments gonflables annulaires (16) au moyen de connexions transversales (18),
**caractérisé en ce que** le moyen de protection comprend un manchon extérieur (20) et un manchon intérieur (22) qui forment un espace (24) qui comprend ladite pluralité de segments gonflables longitudinaux (14) et ladite pluralité de segments gonflables annulaires (16) en forme de cadre de la gaine (10) à l'intérieur de l'espace (24) entre les manchons extérieur et intérieur (20, 22).

2. Moyen de protection selon la revendication 1, dans lequel un segment gonflable annulaire de ladite pluralité de segments gonflables annulaires (16) est situé à l'extrémité distale (10a) de la gaine (10).

3. Moyen de protection selon la revendication 1, dans lequel le manchon extérieur (20) est constitué d'un matériau transparent.

4. Moyen de protection selon la revendication 1, dans lequel le manchon intérieur (22) est constitué d'un matériau transparent.

5. Moyen de protection selon l'une quelconque des revendications précédentes, comprenant en outre des moyens d'injection appropriés pour être connectés au canal gonflable à travers ladite ouverture (15).

6. Moyen de protection selon la revendication 5, dans lequel lesdits moyens d'injection comprennent des moyens pour injecter une solution saline ou un autre fluide biocompatible.

7. Moyen de protection selon l'une quelconque des revendications précédentes, comprenant en outre un dispositif de déploiement (26) qui est approprié pour retenir et insérer ladite gaine (10) à l'état dégonflé jusqu'à l'endroit souhaité du corps.

8. Moyen de protection selon la revendication 7, dans lequel le dispositif de déploiement (26) comprend un corps allongé, et la gaine (10) est appropriée pour être placée et s'agencer sur ledit corps allongé à l'état dégonflé.

9. Moyen de protection selon la revendication 8, dans lequel le corps allongé est transversalement plus petit que la gaine (10) à l'état gonflé, moyennant quoi le corps allongé s'étend à l'intérieur de la gaine à l'état gonflé.

10. Moyen de protection selon la revendication 8 ou 9, dans lequel le corps allongé est un endoscope.
